(19) Europäisches Patentamt
European Patent Office
Office européen des brevets

(11) Veröffentlichungsnummer: **0 507 190 B1**

(12) **EUROPÄISCHE PATENTSCHRIFT**

(45) Veröffentlichungstag der Patentschrift: **21.06.95**

(51) Int. Cl.[6]: **A61K 7/16**, A61K 7/02, A23L 1/226, C07D 317/72, C07D 319/08, C07D 317/20

(21) Anmeldenummer: **92105041.5**

(22) Anmeldetag: **24.03.92**

(54) **Mittel mit physiologischem Kühleffekt und für diese Mittel geeignete wirksame Verbindungen.**

(30) Priorität: **05.04.91 DE 4110973**

(43) Veröffentlichungstag der Anmeldung:
**07.10.92 Patentblatt 92/41**

(45) Bekanntmachung des Hinweises auf die Patenterteilung:
**21.06.95 Patentblatt 95/25**

(84) Benannte Vertragsstaaten:
**AT BE CH DE DK ES FR GB GR IT LI LU NL PT SE**

(56) Entgegenhaltungen:
**EP-A- 0 080 148**
**EP-A- 0 485 170**
**CH-A- 455 832**

**JOURNAL OF THE AMERICAN CHEMICAL SOCIETY, Band 71, Nr. 10, 20. Oktober 1949, Seiten 3303-3307, Washington, DC, US; V. BOEKELHEIDE et al.: "Drugs effecting muscular paralysis. Some substituted dioxolanes and related compounds"**

(73) Patentinhaber: **HAARMANN & REIMER GMBH**
**Postfach 12 53**
**D-37601 Holzminden (DE)**

(72) Erfinder: **Grüb, Helmut, Dr.**
**Dr. Lehmann-Weg 13**
**W-3450 Holzminden (DE)**
Erfinder: **Pelzer, Ralf, Dr.**
**Im Schlossgarten 3**
**W-3476 Fürstenberg (DE)**
Erfinder: **Hopp, Rudolf, Dr.**
**Auf dem Gehrenkamp 28**
**W-3450 Holzminden (DE)**
Erfinder: **Emberger, Roland, Dr.**
**Bärenfang 4**
**W-3450 Holzminden (DE)**
Erfinder: **Bertram, Heinz-Jürgen, Dr.**
**Schneckenbergstrasse 18**
**W-3450 Holzminden (DE)**

(74) Vertreter: **Petrovicki, Wolfgang, Dr. et al**
**Bayer AG**
**Konzernverwaltung RP**
**Patente Konzern**
**D-51368 Leverkusen 1 (Bayerwerk) (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

| | |
|---|---|
| **IL FARMACO, EDIZIONE SCIENTIFICA, Band 35, Nr. 2, Februar 1980, Seiten 89-109, Pavia, IT; G. TAILLANDIER et al.: "Application des paramètres de Verloop. Comparaison avec les autres paramètres stériques, problèmes de leur choix"** | |

## Beschreibung

Die Erfindung betrifft Mittel ohne störenden Eigengeruch und Eigengeschmack, die bei Aufbringen auf die Haut oder auf die Schleimhäute einen physiologischen Kühleffekt bewirken. Offenbar kommt die Wirkung, die der betroffenen Person den Eindruck der Frische vermittelt, durch Stimulierung der entsprechenden Rezeptoren des menschlischen Nervensystems zustande. Weiterhin betrifft die Erfindung neue Verbindungen, die diese Wirkung hervorrufen können.

Die bekannteste natürliche Verbindung mit physiologischer Kühlwirkung ist zweifellos das im Pfefferminzöl (ex Mentha arvensis) vorkommende (-)-Menthol. Es wird beispielsweise für die Herstellung von Zahnreinigungsmitteln, Mundwässern, Nahrungsmitteln, Getränken und Kosmetika eingesetzt. Jedoch werden das typische intensive Pfefferminz-Eigenaroma und der bitter-brennende Eigengeschmack oft als störend empfunden.

Es hat daher nicht an Versuchen gefehlt, Stoffe zu finden, die den positiven Effekt der Kühlwirkung besitzen, ohne die oben geschilderten Nachteile des Menthols aufzuweisen. So werden z.B. in der DE-OS 2 202 535 p-Menthan-3-carboxamid und Ester der p-Menthan-3-carbonsäure, in der DE-OS 2 205 255 N-substituierte p-Menthan-3-carboxamide, in der DE-OS 2 317 538 aliphatische Amide, in der DE-OS 2 334 985 cyclische und acyclische Sulfoxide und Sulfone und in der DE-OS 2 345 156 aliphatische substituierte Phosphinoxide vorgeschlagen. Diese Verbindungen kommen jedoch nicht in der Natur vor und werden vom menschlichen Organismus auch nicht zu natürlich vorkommenden Stoffen abgebaut, so daß sie lebensmittelrechtlich bedenklich sind.

Auch Mentholderivate hat man untersucht; sie erfüllen aber die gestellten Anforderungen nur unvollkommen. So weist das in der DE-OS 2 022 364 vorgeschlagene 1-Menthylethylcarbonat einen orangenartigen Geruch auf, der in der DE-OS 2 433 165 vorgeschlagenen N-Acetylglycin-menthylester sowie die in der DE-OS 2 339 661 vorgeschlagenen Mentholester heterocyclischer Carbonsäuren sind bitter, und die in der US-PS 3 830 930 vorgeschlagenen Menthylketoester sind z.T. anhaltend bitter und weisen nur eine schwache Kühlwirkung auf.

Aus der DE-OS 2 608 226 sind Mentholester natürlich vorkommender $C_2$-$C_6$-Hydroxycarbonsäuren (die gegebenenfalls ihrerseits an der Hydroxygruppe mit $C_1$-$C_4$-Carbonsäurensäuren verestert sind) bekannt, die geruch- und geschmacklos sind und eine langanhaltende Kühlwirkung aufweisen. Insbesondere das 1-Menthyllactat hat sich in der Praxis bewährt. Allerdings ist das Produkt nicht basenstabil, so daß es nicht für alle Anwendungen (z.B. Seifen) geeignet ist.

Auch andere Produkte werden bereits in der Praxis eingesetzt, beispielsweise 3-1-Menthoxypropan-1,2-diol (EP-PS 80 148) und N-Ethyl-p-menthan-3-carboxamid (DE-OS 2 205 255 und 2 413 639). Es bestand jedoch ein Bedarf nach Mitteln mit gesteigerter Kühlwirkung bzw. einem besseren Preis/Leistungs-Verhältnis.

Aus J. Amer. Chem. Soc. 71, 3303-3307(1949) ist bekannt, daß bestimmte Ketale als Muskelrelaxantien wirken.

In Il Farmaco, Ed. Sci., 35, 89-109(1980) werden ebenfalls bestimmte Ketale beschrieben. Der Artikel beschäftigt sich mit sterischen Parametern.

In der CH-A-455 832 werden u.a. Ketale als Ausgangsmaterial für die Herstellung bestimmter Phosphate empfohlen.

Die EP-A 485 170 ist Stand der Technik im Sinne von Art. 54(3) EPÜ. Sie betrifft Verbindungen der Formel

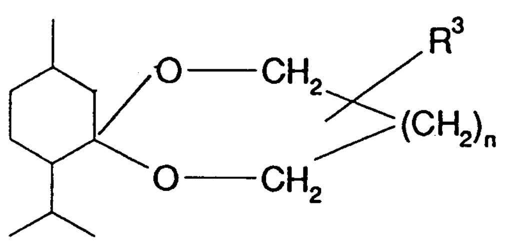

worin $R^3$ Hydroxy, nieder-Alkoxy oder nieder-Alkyl und n Null oder 1 bedeuten,
und die Verwendung dieser Verbindungen als Geschmacksverstärker für verschiedene Lebensmittel und oral anwendbare Mittel, insbesondere für Kaugummi.

Überraschenderweise wurde nun gefunden, daß ausgewählte Ketale die gesuchte glückliche Kombination wünschenswerter Eigenschaften besitzen.

Gegenstand der Erfindung ist die nicht-therapeutische Verwendung von Verbindungen der Formel

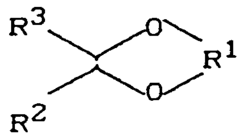

(I)

worin

$R^1$ einen $C_2$-$C_6$-Alkylenrest mit mindestens 1, aber höchstens 3 Hydroxylgruppe(n), und

entweder $R^2$ und $R^3$ unabhängig voneinander $C_1$-$C_{10}$-Alkyl, das gegebenenfalls durch 1 bis 3 Reste aus der Gruppe Hydroxyl, Amino, Halogen substituiert ist, $C_5$-$C_7$-Cycloalkyl, $C_6$-$C_{12}$-Aryl

mit der Maßgabe, daß die Summe der C-Atome von $R^2$ und $R^3$ mindestens 3 beträgt,

oder $R^2$ und $R^3$ zusammen einen Alkylenrest, der zusammen mit dem Kohlenstoffatom, das die Reste $R^2$ und $R^3$ trägt, einen 5-7-gliedrigen Ring bildet, wobei dieser Alkylenrest seinerseits durch $C_1$-$C_6$-Alkylgruppen substituiert sein kann.

darstellen,

zur Erzielung eines Kühleffekts auf menschlicher Haut oder Schleimhaut, mit der Maßgabe für die Vertragsstaaten DE, DK, ES, FR, GB und NL, daß Verbindungen der Formel

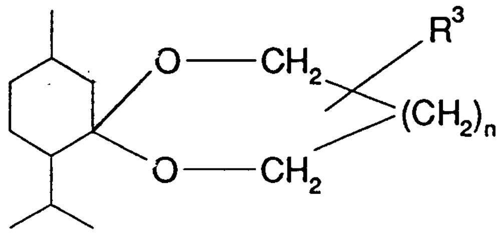

worin $R^3$ Hydroxy, nieder-Alkoxy oder nieder-Alkyl und

n Null oder 1 bedeuten,

bei oraler Verwendung ausgenommen sind.

Bevorzugte Reste $R^2$ und $R^3$ umfassen Methyl, Isopropyl und tert.-Butyl.

Die Länge der Reste $R^2$, $R^3$ beeinflußt die Wirkung der Verbindungen I: Kürzere Reste führen zu einer schnell einsetzenden kurzen Wirkung; längere Reste führen zu einer verzögert einsetzenden, aber lang andauernden Wirkung. Von Bedeutung für die Kosmetikindustrie ist die Wasserlöslichkeit der Verbindungen; diese ist insbesondere bei kurzen Resten $R^2$, $R^3$ gegeben.

Bevorzugte Reste $R^1$ umfassen 1.2- und 1.3-Alkylenreste, die zusammen mit den beiden Sauerstoffatomen und mit dem Kohlenstoffatom, an dem die beiden Sauerstoffatome sitzen, einen Dioxolan- bzw. Dioxanring bilden.

Bevorzugte Verbindungen I, worin $R^2$ und $R^3$ zusammen einen Alkylenrest darstellen, entsprechen der Formel

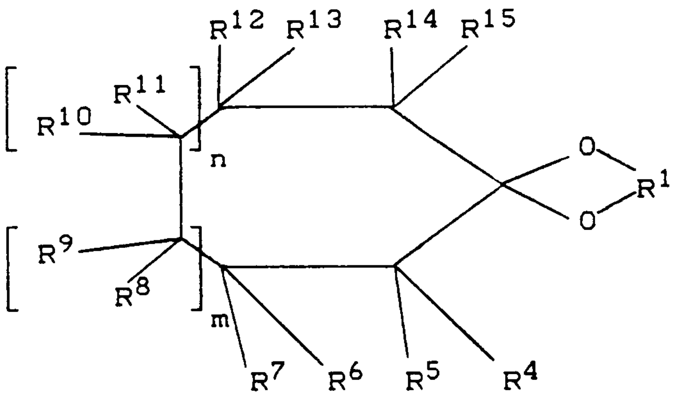

(Ia)

worin

| | |
|---|---|
| $R^4$ und bis $R^{15}$ | unabhängig voneinander Wasserstoff oder $C_1$-$C_6$-Alkyl, vorzugsweise Wasserstoff oder $C_1$-$C_4$-Alkyl, und |
| m, n | unabhängig voneinander Null oder 1 bedeuten. |

Bevorzugte Verbindungen der Formel Ia sind solche, bei denen die Summe m + n 1 ist, also Ketale eines gegebenenfalls substituierten Cyclohexanons.

Bevorzugte Substituenten, von denen insbesondere 1 bis 3 vorhanden sein können, sind Methyl, Isopropyl und tert.-Butyl.

Die Ketale I sind entweder bekannt, oder sie sind bislang unbekannt gewesen und können analog bekannten Verfahren hergestellt werden. So wird man das Ketal I in der Regel durch sauer katalysierte Umsetzung des dem Ketal I zugrundeliegenden Ketons und mindestens der äquivalenten Menge aliphatischem $C_3$-$C_6$-Alkohol mit mindestens 3 und höchstens 5, vorzugsweise mit 3, Hydroxylgruppen herstellen. Im allgemeinen wird man das dem Ketal I zugrundeliegende Keton und mindestens 0,5 Moläquivalente, in der Regel aber das 1.2- bis 4-fache, vorzugsweise das 1.5- bis 3-fache dieser Menge, des $C_3$-$C_6$-Alkohols mit 3 bis 5 Hydroxylgruppen ein setzen. Als sauren Katalysator kann man z.B. p-Toluolsulfonsäure, Phosphorsäure oder Kaliumhydrogensulfat in katalytisch wirksamen Mengen (z.B. 0.1 bis 3 g p-Toluolsulfonsäure pro Mol Keton) verwenden. Vorzugsweise wird man die Reaktion entweder in einem organischen Lösungsmittel durchführen, das mit Wasser ein Azeotrop bildet, so daß das bei der Ketalbildung frei werdende Wasser azeotrop abgeschleppt werden kann, oder man verwendet Wasser-konsumierende Coreagenzien, wie z.B. Trialkylorthoester. Bevorzugte organische Lösungsmittel umfassen z.B. Benzol, Toluol, Xylol, Chloroform, Methylenchlorid und Trichlorethylen. Die Reaktion kann als beendet betrachtet werden, wenn sich kein Wasser mehr abscheidet oder wenn kein Ester/Alkohol-Gemisch mehr abgeschieden wird. Es empfiehlt sich, anschließend mit verdünnter Lauge und mit Wasser zu waschen, die organische Phase abzutrennen und zu trocknen, das Lösungsmittel abzuziehen und den Rückstand gegebenenfalls zu reinigen, z.B. durch Destillation.

Besonders bevorzugte Ketale I entsprechen den Formeln

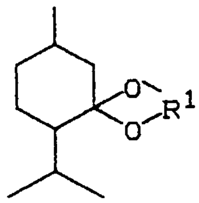

(II)

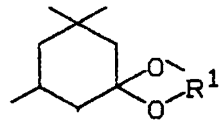

(III)

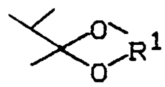

(IV)

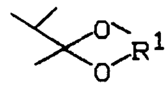

(V)

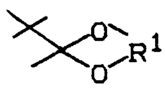

(VI)

worin $R^1$ die oben angegebene Bedeutung hat und vorzugsweise

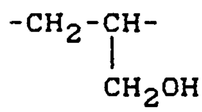 , 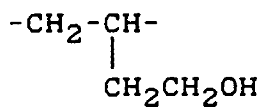 oder 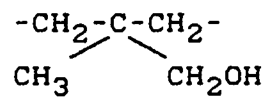

bedeutet. Glycerinketale und deren Homologe werden besonders bevorzugt.

Weiterer Gegenstand der Erfindung sind die Ketale III bis VI.

Die in den erfindungsgemäß herzustellenden Mitteln einzusetzenden Ketale I können asymmetrische C-Atome aufweisen; bei ihnen kann daher optische Isomerie auftreten. Je nach Ausgangsmaterial und angewendeten Herstellungsmethoden können sie als Gemische der optischen Isomeren oder als reine Isomere vorliegen. Die Kühlwirkung der Isomeren kann unterschiedlich sein, so daß das eine oder andere Isomere bevorzugt sein kann.

Die erfindungsgemäß herzustellenden Mittel können neben den Ketalen I Träger und/oder Verdünnungsmittel enthalten. Deren Art richtet sich nach dem vorgesehenen Zweck des Mittels.

Die erfindungsgemäß herzustellenden Mittel können überall da angewendet werden, wo eine physiologische Kühlwirkung erwünscht ist. Zusammensetzungen, in denen solche Kühlmittel gern verwendet werden, sind beispielsweise Genußmittel wie Kaugummi, Kautabak, Zigaretten, Getränke, Eis, Konfekt, Waffelfettmasse, pharmazeutische Präparate, Körperpflegemittel oder kosmetische Präparate, wie Zahnreinigungsmittel, Mundwasser, Gurgelpräparate, Parfüms, Puder, Lotionen, Salben, Oele, Cremes, Rasierschaum, Rasierwässer, Shampoos usw.

Die Endprodukte enthalten die Ketale I in einer Menge, die ausreicht, um das erwünschte Kälteempfinden hervorzurufen. In der Regel kommen 0,01 bis 3, vorzugsweise 0,05 bis 1, Gewichtsprozent, bezogen auf das Gewicht der Gesamtzusammensetzung, zur Anwendung.

Die folgenden Beispiele erläutern die Erfindung. Bei den Prozentangaben handelt es sich, sofern nichts anderes angegeben ist, um Gewichtsprozente.

Beispiele

Beispiel 1: l-Menthonglycerinketal (bekannt aus Svishchuk, A.A.; Makhnovskii, N.K.; Mikryukova, N.Kh. Ukr. Khim. Zh. (Russ. Ed.) 43(2), 173-6, 1977 CA 87(5): 39667u)

In einem 2-l-Dreihalskolben werden 308 g l-Menthon (2 mol), 276 g Glycerin (3 mol) und 5 g Kaliumhydrogensulfat in Toluol vorgelegt. Dieses Gemisch wird am Wasserabscheider unter Rückfluß erhitzt. Nach 7 Stunden sind 42 g Wasser abgeschieden. Nach Neutralisation wird destilliert.

Bei 104-106 °C/1 mbar gehen 441 g einer farblosen klaren Flüssigkeit über. Laut Gaschromatogramm liegt die Reinheit über 99%. Nach dem NMR-Spektrum enthält die resultierende Verbindung einen 1.3-Dioxolanring. $n_D^{20}$ = 1.4749; $\alpha_D^{25}$ = -14.1 °; d(25 °C) = 1.0380.

Beispiel 2: 3,3,5-Trimethylcyclohexanon-glycerinketal

Ein Gemisch aus 140 g 3,3,5-Trimethylcyclohexanon (1 mol), 184 g Glycerin (2 mol) und 2 g p-Toluolsulfonsäure in Toluol wird wie in Beispiel 1 behandelt.

Bei 70-102 °C/1,5 mbar gehen 235 g einer farblosen klaren Flüssigkeit über. Laut Gaschromatogramm beträgt die Reinheit über 99%.
$n_D^{20}$ = 1.4699; d(25 °C) = 1.0369; Fp. < -20 °C.

Beispiele 3-9

3-Methyl-2-butanon-glycerinketal

Ein Gemisch aus 494 g 3-Methyl-2-butanon (5,75 mol), 529 g Glycerin (5,75 mol) und 4 g p-Toluolsulfonsäure wird vorgelegt, anschließend werden 670 g Trimethylorthoformiat (6,32 mol) zugetropft. Dabei steigt die Temperatur des Reaktionsgemisches auf etwa 45 °-50 °C. Das entstandene Methanol-Methylformiat-Gemisch wird bei Normaldruck bis zu einer Sumpftemperatur von 100 °C abdestilliert. Dann wird auf 50 °C abgekühlt, langsam Vakuum angelegt und im Hochvakuum fraktioniert destilliert. Hauptmenge: 716 g; $n_D^{20}$ = 1.4439; d (25 °C) = 1.0294.

Laut Gaschromatogramm beträgt die Reinheit 97,5 %.

Nach dem gleichen Verfahren wurden auch folgende Verbindungen hergestellt:

| Beispiel | $R^2$ | $R^3$ | $n_D^{20}$ | d(25 °C) | Gewicht der Hauptfraktion [g] |
|---|---|---|---|---|---|
| 4 | i-Butyl | Methyl | 1.4426 | 0.9995 | 748 |
| 5 | t-Butyl | Methyl | 1.4486 | 1.0160 | 821 |
| 6 | Ethyl | Ethyl | 1.4447 | 1.0330 | 667 |
| 7 | i-Propyl | i-Propyl | 1.4558 | 1.0167 | 830 |
| 8 | 1,5-Pentylen | | 1.4781 | 1.1117 | 807 |
| 9 | Methyl | Methyl | 1.4341 | 1.0655 | 667 |

Die Beispiele 3-8 betreffen Substanzen mit Kühleffekt; Beispiel 9 zeigt eine Substanz ohne Kühleffekt.

Beispiel 10

1.1.1-Trimethylolethan-ketal des l-Menthons (7-Isopropyl-3,10-dimethyl-1,5-dioxaspiro[5.5]undecan-3-methanol)

Analog Beispiel 1 wurde l-Menthon mit 1.1.1-Trimethylolethan umgesetzt. Man erhielt ein farbloses, sehr zähes Öl;
Kp.: 134 °C (1 mbar);
$n_D^{20}$ : 1,4833; $\alpha_D^{25}$ : -27,3 °C.

Beispiel 11

1.1.1-Trimethylolpropan-ketal des l-Menthons (3-Ethyl-7-isopropyl-10-methyl-1,5-dioxaspiro[5.5]undecan-3-methanol)

Analog Beispiel 1 wurde l-Menthon mit 1.1.1-Trimethylolpropan umgesetzt. Man erhielt ein farbloses, sehr zähes Öl; Kp.: 148 °C (1 mbar);
$n_D^{20}$ : 1,4850; $\alpha_D^{25}$ : -29,1 °C.

Beispiel 12

1.2.4-Butantriol-ketal des l-Menthons (6-Isopropyl-9-methyl-1,4-dioxaspiro[4.5]decan-2-ethanol)

Analog Beispiel 1 wurde l-Menthon mit 1.2.4-Butantriol umgesetzt.

**Patentansprüche**
**Patentansprüche für folgende Vertragsstaaten : DE, DK, ES, FR, GB, NL**

**1.** Nicht-therapeutische Verwendung von Verbindungen der Formel

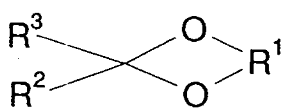

(I)

worin
$R^1$ einen $C_2$-$C_6$-Alkylenrest mit mindestens 1, aber höchstens 3 Hydroxylgruppe(n), und
entweder $R^2$ und $R^3$ unabhängig voneinander $C_1$-$C_{10}$-Alkyl, das gegebenenfalls durch 1 bis 3 Reste aus der Gruppe Hydroxyl, Amino, Halogen substituiert ist, $C_5$-$C_7$-Cycloalkyl, $C_6$-$C_{12}$-Aryl,
mit der Maßgabe, daß die Summe der C-Atome von $R^2$ und $R^3$ mindestens 3 beträgt,
und $R^2$ und $R^3$ zusammen einen Alkylenrest, der zusammen mit dem Kohlenstoffatom, das die Reste $R^2$ und $R^3$ trägt, einen 5-7-gliedrigen Ring bildet, wobei dieser Alkylenrest seinerseits durch $C_1$-$C_6$-Alkylgruppen substituiert sein kann,
darstellen,
zur Erzielung eines Kühleffekts auf menschlicher Haut oder Schleimhaut, mit der Maßgabe, daß Verbindungen der Formel

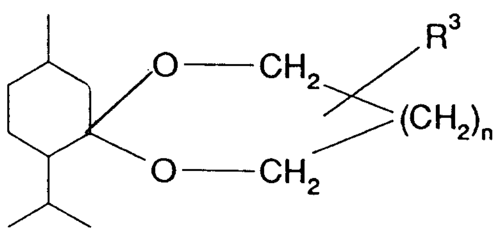

worin $R^3$ Hydroxy, nieder-Alkoxy oder nieder-Alkyl und
n Null oder 1 bedeuten,
bei oraler Verwendung ausgenommen sind.

**2.** Verwendung nach Anspruch 1, wobei $R^1$ einen 1,2- oder 1,3-Alkylenrest bedeutet.

**3.** Verwendung nach Anspruch 1, worin $R^1$

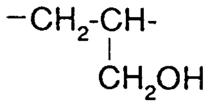 , 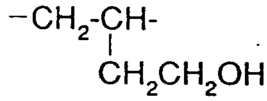 oder 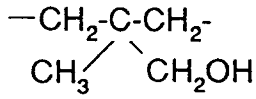

bedeutet.

**4.** Verwendung nach Anspruch 1, wobei das Ketal der Formel

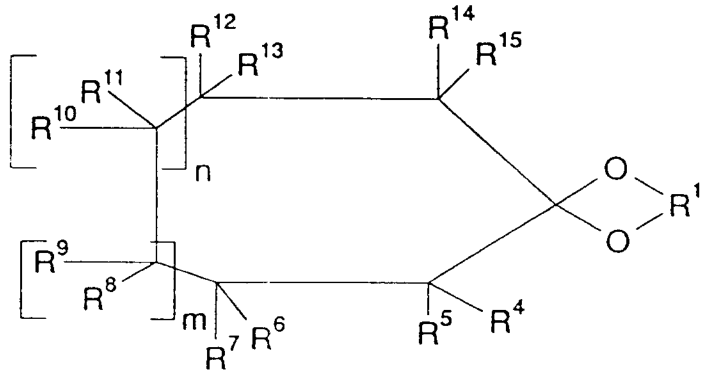 (Ia)

entspricht, worin
$R^1$ die Bedeutung wie in Anspruch 1 hat,
$R^4$ bis $R^{15}$ unabhängig voneinander Wasserstoff oder $C_1$-$C_6$-Alkyl und
m, n unabhängig voneinander Null oder 1 bedeuten.

**5.** Verwendung nach Anspruch 4, wobei die Summe m + n 1 ist.

**6.** Verwendung von Verbindungen der Formel (I) nach Anspruch 1 zur Herstellung eines Mittels zur Erzielung eines Kühleffekts auf menschlicher Haut oder Schleimhaut mit der Maßgabe, daß Verbindungen der Formel

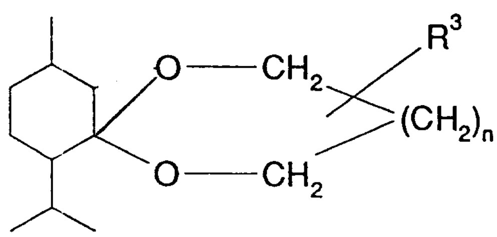

worin $R^3$ Hydroxy, nieder-Alkoxy oder nieder-Alkyl und
n Null oder 1 bedeuten,
ausgenommen sind, wenn es sich um Mittel für orale Verwendung handelt.

**7.** Verbindungen der Formel

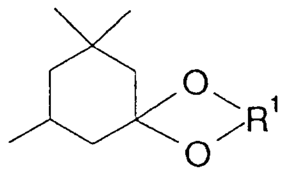 (III)

worin $R^1$ die Bedeutung nach Anspruch **1** hat.

**8.** Verbindung aus der Reihe
3.3.5-Trimethylcyclohexanon-glycerinketal,
1.1.1-Trimethylolethan-Ketal des 1-Menthons,
1.1.1-Trimethylolpropan-Ketal des 1-Menthons,
1.2.4-Butantriol-Ketal des 1-Menthons.

**Patentansprüche für folgende Vertragsstaaten : AT, BE, CH, LI, GR, IT, LU, PT, SE**

**1.** Nicht-therapeutische Verwendung von Verbindungen der Formel

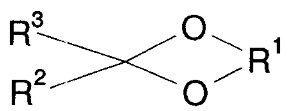 (I)

worin

$R^1$ einen $C_2$-$C_6$-Alkylenrest mit mindestens 1, aber höchstens 3 Hydroxylgruppe(n), und
entweder $R^2$ und $R^3$ unabhängig voneinander $C_1$-$C_{10}$-Alkyl, das gegebenenfalls durch 1 bis 3 Reste aus der Gruppe Hydroxyl, Amino, Halogen substituiert ist, $C_5$-$C_7$-Cycloalkyl, $C_6$-$C_{12}$-Aryl,
mit der Maßgabe, daß die Summe der C-Atome von $R^2$ und $R^3$ mindestens 3 beträgt,
und $R^2$ und $R^3$ zusammen einen Alkylenrest, der zusammen mit dem Kohlenstoffatom, das die Reste $R^2$ und $R^3$ trägt, einen 5-7-gliedrigen Ring bildet, wobei dieser Alkylenrest seinerseits durch $C_1$-$C_6$-Alkylgruppen substituiert sein kann,
darstellen,
zur Erzielung eines Kühleffekts auf menschlicher Haut oder Schleimhaut.

**2.** Verwendung nach Anspruch **1**, wobei $R^1$ einen 1,2- oder 1,3-Alkylenrest bedeutet.

**3.** Verwendung nach Anspruch **1**, worin $R^1$

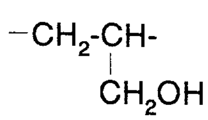 , 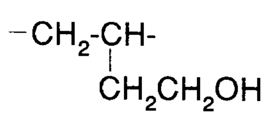 oder 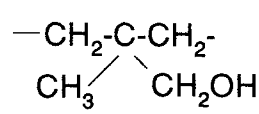

bedeutet.

**4.** Verwendung nach Anspruch 1, wobei das Ketal der Formel

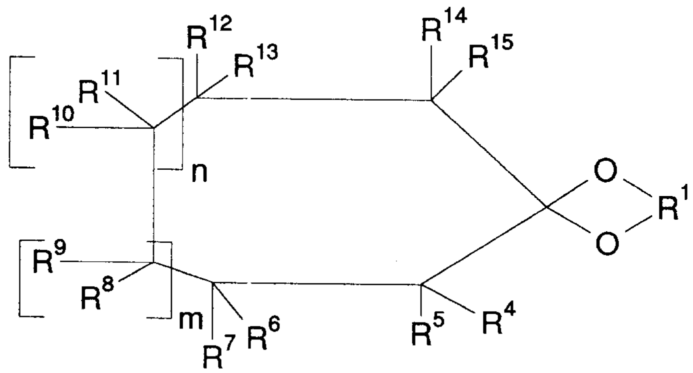

(Ia)

entspricht, worin
$R^1$ die Bedeutung wie in Anspruch 1 hat,
$R^4$ bis $R^{15}$ unabhängig voneinander Wasserstoff oder $C_1$-$C_6$-Alkyl und
m, n unabhängig voneinander Null oder 1 bedeuten.

**5.** Verwendung nach Anspruch 4, wobei die Summe m + n 1 ist.

**6.** Verwendung von Verbindungen der Formel (I) nach Anspruch 1 zur Herstellung eines Mittels zur Erzielung eines Kühleffekts auf menschlicher Haut oder Schleimhaut.

**7.** Verbindungen der Formel

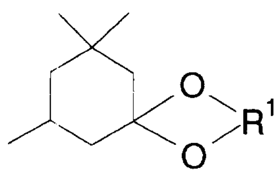

(III)

worin $R^1$ die Bedeutung nach Anspruch 1 hat.

**8.** Verbindung aus der Reihe
3.3.5-Trimethylcyclohexanon-glycerinketal,
1.1.1-Trimethylolethan-Ketal des 1-Menthons,
1.1.1-Trimethylolpropan-Ketal des 1-Menthons,
1.2.4-Butantriol-Ketal des 1-Menthons.

**Claims**
**Claims for the following Contracting States : DE, DK, ES, FR, GB, NL**

**1.** Non-therapeutic use of compounds of the formula

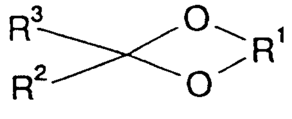

(I)

in which

$R^1$ represents a $C_2$-$C_6$-alkylene radical having at least 1, but not more than 3, hydroxyl group(s), and

either $R^2$ and $R^3$ independently of one another represent $C_1$-$C_{10}$-alkyl which is optionally substituted by 1 to 3 radicals from the group comprising hydroxyl, amino and halogen, or $C_5$-$C_7$-cycloalkyl, or $C_6$-$C_{12}$-aryl,

with the proviso that the total of the C atoms of $R^2$ and $R^3$ is not less than 3,

or $R^2$ and $R^3$ together represent an alkylene radical which, together with the carbon atom which carries the radicals $R^2$ and $R^3$,

forms a 5-7-membered ring, it being possible for this alkylene radical, for its part, to be substituted by $C_1$-$C_6$-alkyl groups,

for achieving a cooling effect on human skin or mucous membrane, with the proviso that compounds of the formula

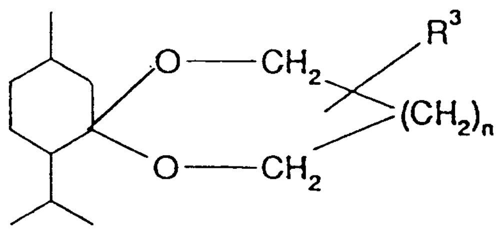

in which

$R^3$ is hydroxyl, lower alkoxy or lower alkyl and

n is zero or 1,

are excluded in oral use.

2. Use according to Claim 1, where $R^1$ denotes a 1,2- or 1,3-alkylene radical.

3. Use according to Claim 1, where $R^1$ denotes

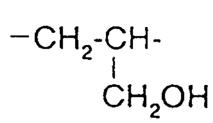 , 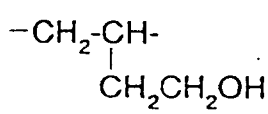 or 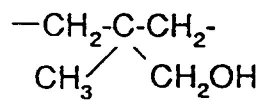

4. Use according to Claim 1, where the ketal corresponds to the formula

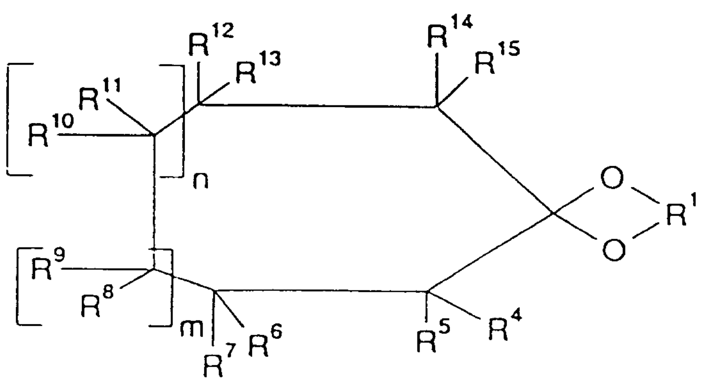 (Ia)

in which
$R^1$ is as defined in Claim 1,
$R^4$ to $R^{15}$ independently of one another denote hydrogen or $C_1$-$C_6$-alkyl and
m and n independently of one another denote zero or 1.

**5.** Use according to Claim 4, where the total of m + n is 1.

**6.** Use of compounds of the formula (I) according to Claim 1 for the preparation of a composition for achieving a cooling effect on human skin or mucous membrane, with the proviso that compounds of the formula

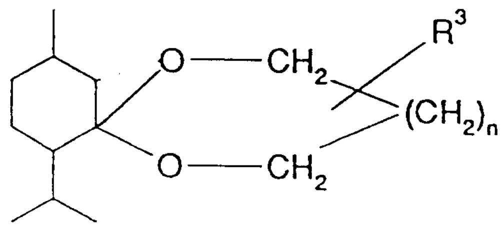

in which $R^3$ is hydroxyl, lower alkoxy or lower alkyl and
n is zero or 1,
are excluded if the compositions are for oral use.

**7.** Compounds of the formula

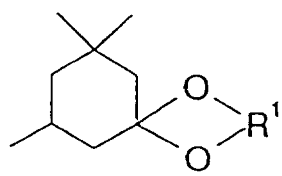 (III)

in which $R^1$ is as defined in Claim 1.

**8.** Compound from the series comprising
3,3,5-trimethylcyclohexanone glycerol ketal,
1,1,1-trimethylolethane ketal of 1-menthone,
1,1,1-trimethylolpropane ketal of 1-menthone,
1,2,4-butanetriol ketal of 1-menthone.

**Claims for the following Contracting States : AT, BE, CH, LI, GR, IT, LU, PT, SE**

**1.** Non-therapeutic use of compounds of the formula

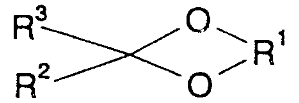 (I)

in which
$R^1$ represents a $C_2$-$C_6$-alkylene radical having at least 1, but not more than 3, hydroxyl group(s), and
either $R^2$ and $R^3$ independently of one another represent $C_1$-$C_{10}$-alkyl which is optionally substituted by 1 to 3 radicals from the group comprising hydroxyl, amino and halogen, or $C_5$-$C_7$-cycloalkyl, or $C_6$-$C_{12}$-

aryl,

with the proviso that the total of the C atoms of $R^2$ and $R^3$ is not less than 3,

or $R^2$ and $R^3$ together represent an alkylene radical which, together with the carbon atom which carries the radicals $R^2$ and $R^3$, forms a 5-7-membered ring, it being possible for this alkylene radical, for its part, to be substituted by $C_1$-$C_6$-alkyl groups,

for achieving a cooling effect on human skin or mucous membrane.

**2.** Use according to Claim 1, where $R^1$ denotes a 1,2- or 1,3-alkylene radical.

**3.** Use according to Claim 1, where $R^1$ denotes

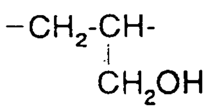 , 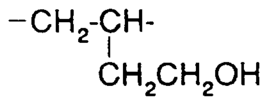 or 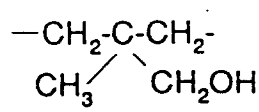

**4.** Use according to Claim 1, where the ketal corresponds to the formula

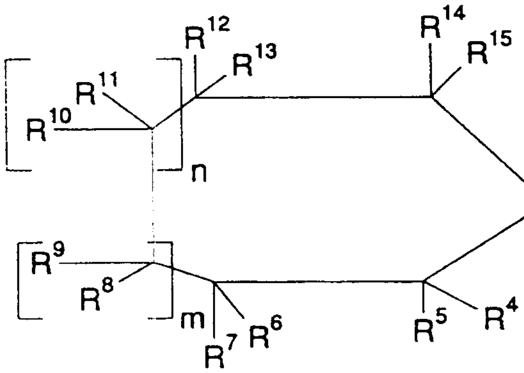 (Ia)

in which

$R^1$ is as defined in Claim 1,

$R^4$ to $R^{15}$ independently of one another denote hydrogen or $C_1$-$C_6$-alkyl and

m and n independently of one another denote zero or 1.

**5.** Use according to Claim 4, where the total of m + n is 1.

**6.** Use of compounds of the formula (I) according to Claim 1 for the preparation of a composition for achieving a cooling effect on human skin or mucous membrane.

**7.** Compounds of the formula

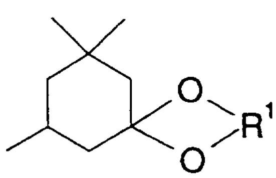 (III)

in which $R^1$ is as defined in Claim 1.

8. Compound from the series comprising
3,3,5-trimethylcyclohexanone glycerol ketal,
1,1,1-trimethylolethane ketal of 1-menthone,
1,1,1-trimethylolpropane ketal of 1-menthone,
1,2,4-butanetriol ketal of 1-menthone.

**Revendications**
**Revendications pour les Etats contractants suivants : DE, DK, ES, FR, GB, NL**

1. Utilisation non thérapeutique de composés de formule :

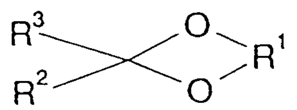

(I)

dans laquelle
$R^1$ représente un reste alkylène en $C_2$-$C_6$ avec au moins un et au plus trois groupes hydroxyle, et
soit $R^2$ et $R^3$ représentent indépendamment l'un de l'autre un alkyle en $C_1$-$C_{10}$, qui peut être substitué le cas échéant par 1 à 3 restes du groupe hydroxyle, amino, halogène, un cycloalkyle en $C_5$-$C_7$, un aryle en $C_6$-$C_{12}$, à la condition que la somme des atomes de C de $R^2$ et $R^3$ représente au moins 3,
soit $R^2$ et $R^3$ représentent ensemble un reste alkylène, qui forme avec l'atome de carbone qui porte les restes $R^2$ et $R^3$, un cycle à 5-7 maillons, ce reste alkylène pouvant à son tour être substitué par des groupes alkyle en $C_1$-$C_6$, pour obtenir un effet rafraîchissant sur la peau humaine ou les muqueuses, à la condition que les composés de formule

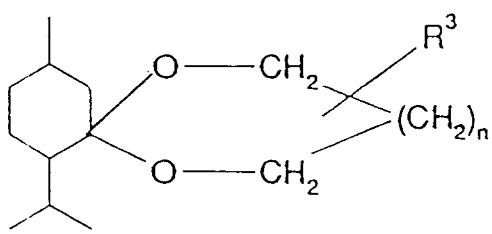

dans laquelle $R^3$ est un hydroxy, un alcoxy inférieur ou un alkyle inférieur et n est 0 ou 1, soient exclus de l'utilisation orale.

2. Utilisation selon la revendication 1, où $R^1$ représente un reste 1,2 ou 1,3-alkylène.

3. Utilisation selon la revendication 1, où $R^1$ représente

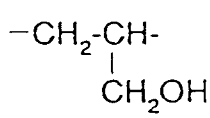 , 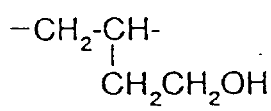 ou 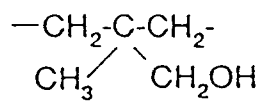

**4.** Utilisation selon la revendication 1 où l'acétal répond à la formule :

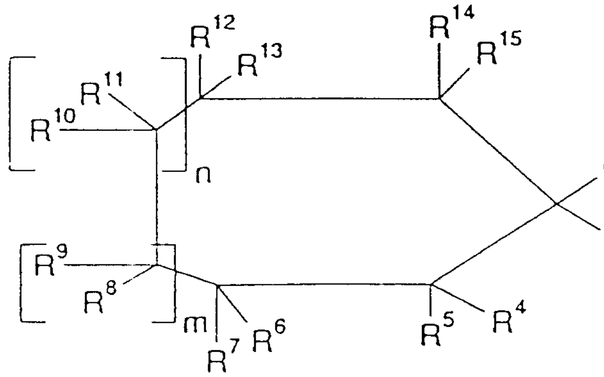

(Ia)

dans laquelle $R^1$ a la même signification qu'à la revendication 1,
$R^4$ à $R^{15}$ représentent indépendamment l'un de l'autre un hydrogène ou un alkyle en $C_1$-$C_6$ et m, n représentent indépendamment l'un de l'autre 0 ou 1.

**5.** Utilisation selon la revendication 4, où la somme m + n est 1.

**6.** Utilisation de composés de formule (I) selon la revendication 1 pour préparer un moyen destiné à obtenir un effet rafraîchissant sur la peau humaine ou les muqueuses à la condition que les composés de formule :

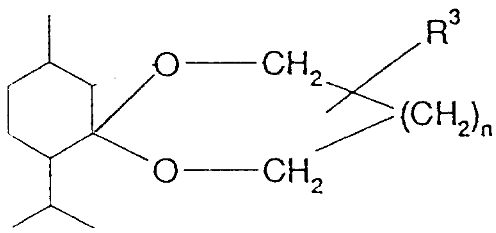

dans laquelle $R^3$ représente un hydroxy, un alcoxy inférieur ou un alkyle inférieur et n représente 0 ou 1,
sont exclus, quand il s'agit d'un moyen d'utilisation orale.

**7.** Composés de formule :

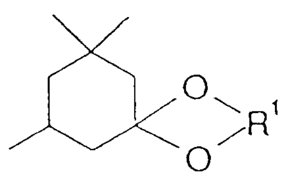

(III)

dans laquelle $R^1$ a la signification selon la revendication 1.

**8.** Composé de la série
cétal glycérique de la 3,3,5-triméthylcyclohexanone,
cétal 1,1,1-triméthyloléthylique de la 1-menthone,
cétal 1,1,1-triméthylolpropylique de la 1-menthone,
cétal 1,2,4-butanetriolique de la 1-menthone.

**Revendications pour les Etats contractants suivants : AT, BE, CH, LI, GR, IT, LU, PT, SE**

1. Utilisation non thérapeutique de composés de formule :

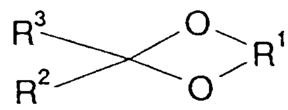

(I)

dans laquelle

$R^1$ représente un reste alkylène en $C_2$-$C_6$ avec au moins un et au plus trois groupes hydroxyle, et

soit $R^2$ et $R^3$ représentent indépendamment l'un de l'autre un alkyle en $C_1$-$C_{10}$, qui peut être substitué le cas échéant par 1 à 3 restes du groupe hydroxyle, amino, halogène, un cycloalkyle en $C_5$-$C_7$, un aryle en $C_6$-$C_{12}$, à la condition que la somme des atomes de C de $R^2$ et $R^3$ représente au moins 3,

soit $R^2$ et $R^3$ représentent ensemble un reste alkylène, qui forme avec l'atome de carbone qui porte les restes $R^2$ et $R^3$, un cycle à 5-7 maillons, ce reste alkylène pouvant à son tour être substitué par des groupes alkyle en $C_1$-$C_6$,

pour obtenir un effet rafraîchissant sur la peau humaine ou les muqueuses.

2. Utilisation selon la revendication 1, où $R^1$ représente un reste 1,2 ou 1,3-alkylène.

3. Utilisation selon la revendication 1, où $R^1$ représente

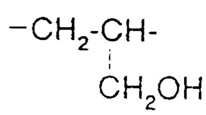 , 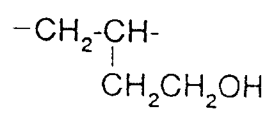 ou 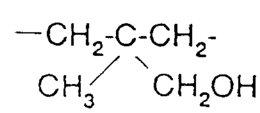

4. Utilisation selon la revendication 1 où l'acétal répond à la formule :

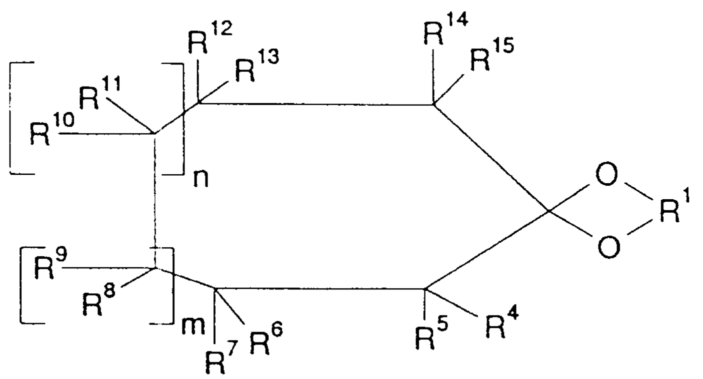

(Ia)

dans laquelle $R^1$ a la même signification qu'à la revendication 1,

$R^4$ à $R^{15}$ représentent indépendamment l'un de l'autre un hydrogène ou un alkyle en $C_1$-$C_6$ et m, n représentent indépendamment l'un de l'autre 0 ou 1.

5. Utilisation selon la revendication 4, où la somme m + n est 1.

6. Utilisation de composés de formule (I) selon la revendication 1 pour préparer un moyen destiné à obtenir un effet rafraîchissant sur la peau humaine ou les muqueuses.

**7.** Composés de formule :

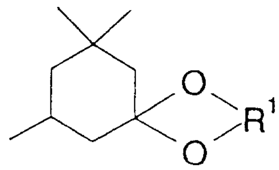

(III)

dans laquelle $R^1$ a la signification selon la revendication 1.

**8.** Composé de la série
cétal glycérique de la 3,3,5-triméthylcyclohexanone,
cétal 1,1,1-triméthyloléthylique de la 1-menthone,
cétal 1,1,1-triméthylolpropylique de la 1-menthone,
cétal 1,2,4-butanetriolique de la 1-menthone.